# EUROPÄISCHE PATENTANMELDUNG

(11) **EP 2 944 328 A2**
(43) Veröffentlichungstag der Anmeldung: **18.11.2015**
(21) Anmeldenummer: 15163009.2
(22) Anmeldetag: 09.04.2015
(51) Int. Cl.: A61L 2/20, A61L 2/26

(54) **VERPACKUNGSEINHEIT FÜR EIN PHARMAZEUTISCHES, MEDIZINISCHES ODER KOSMETISCHES OBJEKT UND VERFAHREN ZUM STERILISIEREN EINES IN DER VERPACKUNGSEINHEIT ANORDENBAREN PHARMAZEUTISCHEN, MEDIZINISCHEN ODER KOSMETISCHEN OBJEKTS**

(30) Priorität: 24.04.2014 DE 102014105787
(71) Anmelder: Schott AG, 55122 Mainz (DE)
(72) Erfinder: Pawlowski, Edgar, 55271 Stadecken-Elsheim (DE); Deutschle, Gregor Fritz, 65185 Wiesbaden (DE); Dubrau, Isabell, 9000 St. Gallen (CH); Wassenberg, Jörn, 55130 Mainz (DE); Auerbach, Judith, 9052 Niederteufen (CH)
(74) Vertreter: Blumbach Zinngrebe

(57) **Zusammenfassung**

Die Erfindung betrifft eine Verpackungseinheit (10) für mindestens ein pharmazeutisches, medizinisches oder kosmetisches Objekt (12). Die Verpackungseinheit (10) umfasst:
eine Sterilisationskammer (14) zum Aufnehmen des Objekts (12) und
eine Vorkammer (16), die zu einem Außenbereich der Verpackungseinheit (10) und zu der Sterilisationskammer (14) hin jeweils mehrfach verschließbar ist,
wobei eine Wandung der Vorkammer (16) bereichsweise ein Wandmaterial (18) mit selektiver Durchlässigkeit für Sterilisationsmittel (24, 26) umfasst.

## Beschreibung

### Gebiet der Erfindung

Die Erfindung betrifft eine Verpackungseinheit für ein pharmazeutisches, medizinisches oder kosmetisches Objekt, insbesondere einen Verpackungsbehälter für mindestens einen pharmazeutischen Behälter. Die Erfindung betrifft ferner eine Verpackungseinheit einschließlich des darin angeordneten pharmazeutischen, medizinischen oder kosmetischen Objekts, eine Verwendung der Verpackungseinheit zum Sterilisieren und sterilen Transportieren des pharmazeutischen Objekts, und ein Verfahren zum Sterilisieren eines in der Verpackungseinheit anordenbaren pharmazeutischen, medizinischen oder kosmetischen Objekts.

### Hintergrund der Erfindung

Als Behälter zur Aufbewahrung und Lagerung von medizinischen, pharmazeutischen oder kosmetischen Präparaten mit Verabreichung in flüssiger Form, insbesondere in vordosierten Mengen, werden in großem Umfang Behälter, wie beispielsweise Fläschchen, Ampullen oder Karpulen, eingesetzt. Diese weisen generell eine zylindrische Form auf und können aus Kunststoff oder Glas hergestellt werden. Für eine möglichst wirtschaftliche Befüllung der Behälter unter sterilen Bedingungen werden die Behälter beim Hersteller in einem Transport- und Verpackungsbehälter steril verpackt. Anschließend, bei einem Pharmaunternehmen, werden sie unter sterilen Bedingungen, insbesondere in einem Steriltunnel, ausgepackt und befüllt.

Für den Transport der Transportbehälter werden oft gasdichte Transportbeutel verwendet. Mit den bekannten Transportbeuteln ist es allerdings schwierig, die Transportbehälter außen und innen keimfrei zu halten und während der Aufbewahrung oder des Transportes die Keimfreiheit aufrechtzuerhalten.

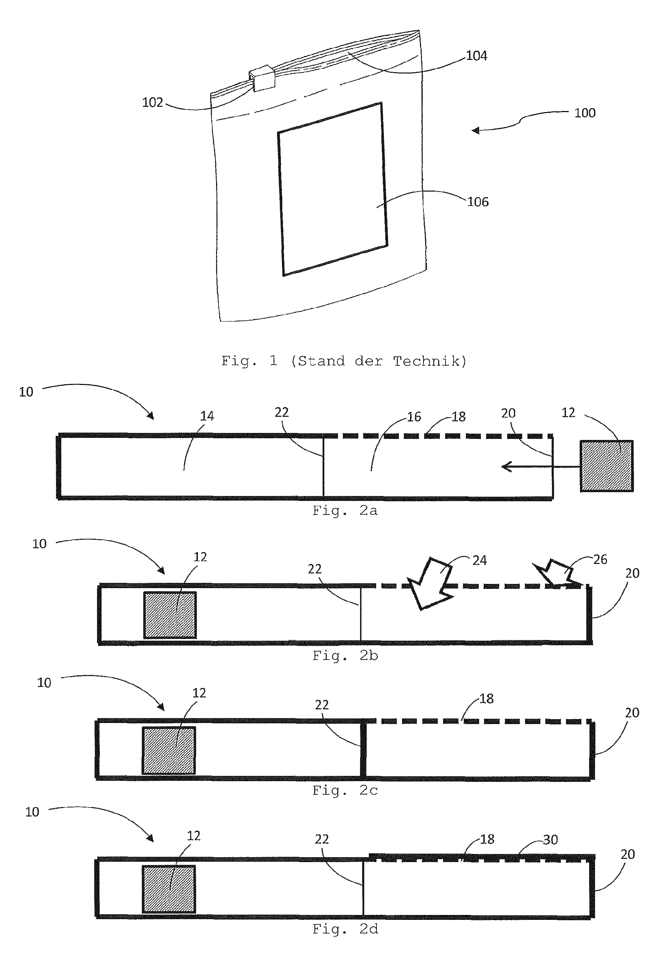

Aus dem Stand der Technik ist ein aus einer Plastik-Folie bestehender Schieberbeutel 100 bekannt (Fig. 1), der eine mittels eines Schieber-Reißverschlusses 102 schließbare Öffnung 104 aufweist. Durch die Öffnung 104 kann ein pharmazeutisches Objekt wie beispielsweise ein Medikamentenbehälter in den Schieberbeutel 100 eingeführt werden und anschließend kann der Schieber-Reißverschluss 102 geschlossen werden. Durch die Öffnung 104 können der Innenbereich des Schieberbeutels 100 und das darin angeordnete Objekt dekontaminiert werden. Ein beschrifteter Aufkleber 106 gibt Auskunft über das in dem Schieberbeutel 100 enthaltene Objekt.

Eine Behandlung des Außen- und Innen- Bereichs des Schieberbeutels 100 mit unterschiedlichen Sterilisationsmitteln ist allerdings mit einem relativ hohen Aufwand verbunden. Ferner kann, nach einer Innenbereich-Sterilisierung, beim Betätigen des Schieber-Reißverschlusses 102 zum Verschließen der Öffnung 104 der Innenbereich erneut mit Mikroorganismen kontaminiert werden. Eine ähnliche Kontaminierungsgefahr besteht auch bei Verwendung eines Vakuumierers zum Evakuieren des Schieberbeutels 100. Darüber hinaus ist bei Verwendung des Vakuumierers und einer damit verbundenen Folienschweißung eine Wiederverwendung des Schieberbeutels 100 nicht möglich.

### Allgemeine Beschreibung der Erfindung

Der Erfindung liegt die Aufgabe zugrunde, eine Verpackungseinheit für ein pharmazeutisches, medizinisches oder kosmetisches Objekt bereit zu stellen, welche die erwähnten Nachteile des Stands der Technik überwindet. Insbesondere soll eine Verpackungseinheit bereitgestellt werden, die eine einfache und effiziente Erzielung oder Bewahrung einer hohen Sterilität des Objekts ermöglicht.

Diese Aufgabe wird durch den Gegenstand der unabhängigen Ansprüche gelöst. Vorteilhafte Ausgestaltungen und Weiterbildungen sind in den abhängigen Ansprüchen angegeben. Die Merkmale der Weiterbildungen können, soweit technisch sinnvoll, miteinander kombiniert werden.

Im vorliegenden Dokument wird die ODER-Verknüpfung als eine nicht-ausschließende Disjunktion aufgefasst. Demnach ist die Verknüpfung "A oder B" dann wahr, wenn mindestens eine der beteiligten Aussagen A, B wahr ist.

Ein Aspekt der Erfindung betrifft eine Verpackungseinheit für ein pharmazeutisches Objekt. Die Verpackungseinheit kann ein Transport- und Verpackungsbehälter sein, in welchem das pharmazeutische Objekt vorzugsweise steril verpackt, gelagert oder transportiert werden kann. Das pharmazeutische Objekt kann i) ein pharmazeutischer oder medizinischer Behälter oder ii) ein Verpackungsbehälter, Behälternest oder Tub für mindestens einen Behälter sein. Der Behälter kann vorzugsweise ein Medikamentenbehälter, ein Fläschchen, eine Ampulle, eine Karpule, eine Vartridge, eine Doppelkammer-Karpule, eine Spritze, oder eine Doppelkammer-Spritze sein.

Die Verpackungseinheit kann eine Sterilisationskammer zum Aufnehmen des Objekts und eine Vorkammer umfassen und kann damit eine Zwei-Kammer-Struktur aufweisen. Die Vorkammer kann zu einem Außenbereich der Verpackungseinheit und zu der Sterilisationskammer hin jeweils verschließbar sein. Das heißt, die Vorkammer kann zu dem Außenbereich und zu der Sterilisationskammer hin Öffnungen aufweisen, die jeweils mittels eines, vorzugsweise wiederzuöffnenden Verschlusses oder mittels Verschweißen verschließbar sind.

Durch die Öffnungen kann das Objekt in die Sterilisationskammer eingebracht werden, wobei nach dem Einbringen des Objekts der Verschluss zu dem Außenbereich verschlossen werden kann, um eine Kontaminierung des Innenbereichs mit Mikroorganismen zu verhindern und um den Start einer Sterilisation des Innenbereichs zu ermöglichen.

Eine Wandung der Vorkammer kann bereichsweise ein Wandmaterial mit selektiver Durchlässigkeit für Sterilisationsmittel umfassen. Hierbei kann die selektive Durchlässigkeit bedeuten, dass das Wandmaterial für ein, insbesondere gasförmiges, Sterilisationsmittel durchlässig ist. Allgemein werden für das Wandmaterial Textilstoffe bevorzugt, wobei unter den Begriff eines Textilstoffes Gewebe, Gewirke und insbesondere auch Vliesstoffe fallen.

In dem vorliegenden Dokument wird unter Sterilisieren generell das Sterilisieren, Desinfizieren, oder Dekontaminieren verstanden. Vorzugsweise kann ein erstes Sterilisationsmittel, beim Beströmen der Verpackungseinheit mit dem ersten Sterilisationsmittel, durch das Wandmaterial in einen Innenbereich der Verpackungseinheit durchdringen um den Innenbereich zu Sterilisieren. Zugleich kann das Wandmaterial, beim Beströmen der Verpackungseinheit mit einem zweiten Sterilisationsmittel, z.B. mit der Wasserstoffperoxidtechnologie, realisiert werden.
Beim Sterilisieren sollte eine Keimreduktion von mindestens vier Zehnerpotenzen (bzw. sechs Zehnerpotenzen für bestimmte Anwendungen) eines bestimmten und für das Entkeimungsverfahren geeigneten Testkeims erreicht werden.
Beispielsweise ist bei der Verwendung der Wasserstoffperoxidtechnologie der Bacillus subtilis SA22 als Testkeim einzusetzen.

Das zweite Sterilisationsmittel kann wirkungsstärker und damit für lebende Organismen toxischer als das erste Sterilisationsmittel sein. Auf dieser Basis wird eine schnellere und wirkungsstärkere Sterilisation des Außenbereichs als die des Innenbereichs erreicht.
Auch kann für den Außenbereich ein Sterilisationsmittel verwendet werden, welches die Behälter im Inneren nicht kontaminiert.

Das Wandmaterial mit selektiver Durchlässigkeit ermöglicht vorteilhafter Weise eine strenge Wahrung der Sterilität in dem Innenbereich der Verpackungseinheit oder der Sterilisationskammer: nach dem Einbringen des Objekts in die Verpackungseinheit und dem Verschließen der Vorkammer-Öffnung zu dem Außenbereich können Mikroorganismen nicht mehr in die Verpackungseinheit gelangen. Nach einer Sterilisation des Innenbereichs mit dem ersten Sterilisationsmittel bleibt somit der Innenbereich oder das Objekt steril. Die Gefahr einer anschließenden Kontaminierung des Innenbereichs mit Mikroorgansimen ist gleich Null, solange die Vorkammer-Öffnung zu dem Außenbereich verschlossen bleibt.

Die Zwei-Kammer-Struktur der Verpackungseinheit ermöglicht vorteilhafter Weise, nach dem Verschließen der Vorkammer-Öffnung zu der Sterilisationskammer, das Evakuieren der Sterilisationskammer, insbesondere auf einen Druck von < 200 mbar. Der Unterdruck stellt eine weitere, erhebliche Verbesserung der Sterilität in der Sterilisationskammer dar. Hierbei bedeutet eine verbesserte Sterilität eine weitere Reduktion der Anzahl von Mikroorganismen. Mit der vorliegenden Verpackungseinheit ist also eine zweistufige Reduktion der Anzahl von Mikroorganismen möglich: i) mittels Sterilisieren, wobei Mikroorganismen weitgehend abgetötet werden, und ii) mittels Evakuieren, wobei ein Großteil der im Gas übriggebliebenen Mikroorganismen abgepumpt werden. Durch das Evakuieren wird auch die Dichtheit des Beutels auf einfachere Weise indiziert.

Ein weiterer Aspekt der Erfindung betrifft die oben geschilderte Verpackungseinheit mit einem pharmazeutischen Objekt, welches als ein Behälter oder ein Verpackungsbehälter, Behälternest oder Tub für mindestens einen Behälter ausgebildet ist. Der Behälter ist vorzugsweise ein Medikamentenbehälter, ein Fläschchen, eine Ampulle, eine Karpule, eine Vartridge, eine Doppelkammer-Karpule, eine Spritze, oder eine Doppelkammer-Spritze.

Ein weiterer Aspekt der Erfindung betrifft eine Verwendung der oben geschilderten Verpackungseinheit zum Sterilisieren und sterilen Transportieren eines pharmazeutischen Objekts, welches als i) ein Behälter oder ii) ein Verpackungsbehälter, Behälternest oder Tub für mindestens einen Behälter ausgebildet ist. Der Behälter ist vorzugsweise ein Medikamentenbehälter, ein Fläschchen, eine Ampulle, eine Karpule, eine Vartridge, eine Doppelkammer-Karpule, eine Spritze, oder eine Doppelkammer-Spritze.

Ein Aspekt der Erfindung betrifft ein Verfahren zum Sterilisieren eines in einer Verpackungseinheit anordenbaren pharmazeutischen Objekts. Die Verpackungseinheit kann eine Sterilisationskammer und eine mit der Sterilisationskammer fluidverbundene Vorkammer, die zu einem Außenbereich der Verpackungseinheit verschließbar geöffnet ist und eine Wandung aufweist, die bereichsweise ein Wandmaterial mit selektiver Durchlässigkeit für Sterilisationsmittel umfasst.

Das Verfahren kann die Schritte umfassen: i) Einbringen des Objekts in die Sterilisationskammer, ii) Verschließen der Vorkammer gegenüber dem Außenbereich, und iii) Einleiten eines ersten Sterilisationsmittels, für welches das Wandmaterial durchlässig ist, von dem Außenbereich durch das Wandmaterial in einen Innenbereich der Verpackungseinheit, zum Sterilisieren des Innenbereichs oder des Objekts.

Gemäß einer Ausführungsform kann das selektiv durchlässige Wandmaterial für ein, insbesondere gasförmiges, erstes Sterilisationsmittel durchlässig sein, wobei vorzugsweise das erste Sterilisationsmittel durch das Wandmaterial in einen Innenbereich der Verpackungseinheit zum Sterilisieren des Innenbereichs einleitbar ist.

Vorzugsweise kann das selektiv durchlässige Wandmaterial für Mikroorganismen undurchlässig sein.

Das erste Sterilisationsmittel kann ein als ETO bezeichnetes Ethylenoxid umfassen oder sein. Das zweite Sterilisationsmittel kann einen als VHP bezeichneten Wasserstoffperoxiddampf umfassen oder sein.

Das selektiv durchlässige Wandmaterial kann einen textilen Stoff, wie insbesondere einen Vliesstoff umfassen, der vorzugsweise aus oder mit Kunststofffasern gebildet ist. Insbesondere sind textile Stoffe aus oder mit hydrophoben Kunststofffasern geeignet. Geeignete Kunststoffe für das selektiv durchlässige Wandmaterial sind dabei besonders Polyethylen, Polypropylen und PET.

Ein sehr geeignetes Wandmaterial ist ein Polyethylen-Vliesstoff. Als Beispiel sei der Vliesstoff Tyvek® genannt. Tyvek® ist eine registrierte Marke der Firma DuPont für einen Vliesstoff aus Polyethylen hoher Dichte (PE-HD), der sich aus fibrillierten, eng miteinander zu Netzwerken verbundenen Feinstfilamenten im Durchmesserbereich von 0,5 bis 10 µm aufbaut. Auf der Basis von Tyvek® können alle gängigen Sterilisationsverfahren verwendet werden, einschließlich ETO, Gamma- und Elektronenstrahlen, Dampf (unter kontrollierten Bedingungen) sowie die Wasserstoffperoxid-Plasma Sterilisation. Tyvek® ermöglicht es, dass Sterilisationsgase und Dampf schnell eindringen und wieder entweichen können. Unabhängig vom Sterilisationsverfahren bietet Tyvek® einen Schutz als mikrobielle Barriere und bewahrt seine Festigkeit.

Die Vorkammer kann
- einen ersten, insbesondere gasdichten, wiederverschließbaren Verschluss zum, vorzugsweise öffnenbaren, Verschließen der Öffnung zu dem Außenbereich aufweisen, sowie
- einen zweiten, insbesondere gasdichten, wiederverschließbaren Verschluss zum, vorzugsweise öffnenbaren, Verschließen der Öffnung zu der Sterilisationskammer aufweisen.

Der erste oder zweite Verschluss kann von außen betätigbar sein zum Schließen oder Öffnen der entsprechenden Öffnungen. Dadurch kann vorteilhafter Weise eine Kontaminierung des Innenbereichs mit Mikroorganismen durch das Bedienen der Verschlüsse verhindert werden.

Der erste oder zweite Verschluss kann als ein Druckverschluss, Reißverschluss, oder Ziplock, vorzugsweise als ein einfacher oder mehrfacher Verschluss, ausgebildet sein. Hierbei ist Ziplock eine Marke für einen wiederverwendbaren, wiederverschließbaren Reißverschluss für Aufbewahrungsbeutel und Behälter, der ursprünglich von Dow Chemical entwickelt und vermarktet wurde.

Die Verpackungseinheit kann als ein hohler, dünnwandiger, leicht verformbarer Gegenstand, vorzugsweise als ein Beutel, ausgebildet sein. Eine Wandung der Verpackungseinheit kann resistent gegen VHP oder ETO sein und kann vorzugsweise einen chemikalienresistenten Kunststoff umfassen, so dass der Beutel wieder verwendbar ist. Der Kunststoff kann ein als PA bezeichnetes Polyamid oder ein als PE bezeichnetes Polyethylen oder ein als PC bezeichnetes Polycarbonat oder ein als PP bezeichnetes Polypropylen oder ein als PSU bezeichnetes Polysulfon oder ein als PVC bezeichnetes Polyvinylchlorid sein.

Die Wandung der Verpackungseinheit kann eine Folie aus einem Metall, vorzugsweise Aluminium, oder aus einem Aluminium-Polypropylen-Verbundmaterial umfassen. Damit kann eine verbesserte Steifigkeit und Chemikalienresistenz des Beutels erreicht werden.

Die Verpackungseinheit, insbesondere die Sterilisationskammer, kann evakuierbar sein, wobei vorzugsweise ein im Inneren der Verpackungseinheit oder der Sterilisationskammer vorhandenes Gas abpumpbar ist zum Reduzieren eines Drucks im Inneren der Verpackungseinheit oder der Sterilisationskammer. Dazu kann an der Sterilisationskammer ein Vakuumport vorgesehen sein, an welchen eine Vakuumpumpe anschließbar ist.

Gemäß einer Ausführungsform kann zumindest ein physikalischer Parameter im Inneren der Verpackungseinheit, insbesondere eine Temperatur, Gaszusammensetzung, Feuchte, Druck, oder eine elektromagnetische Strahlung messbar sein oder gemessen werden, vorzugsweise mittels zumindest eines in der Verpackungseinheit angeordneten Sensors. Als elektromagnetische Strahlung ist vorzugsweise UV-Strahlung oder auch Gammastrahlung gemeint. Zur besseren Messung der UV-Strahlung kann vorzugsweise ein UV-transparentes Fenster verwendet werden.

Die Verpackungseinheit kann zumindest ein Mittel zum Ändern des physikalischen Parameters umfassen, beispielsweise Heiz- oder Kühl- Elemente, oder an ein solches Mittel angeschlossen werden. So kann die Sterilisationskammer über ein Vakuumport an eine externe Vakuumpumpe anschließbar sein. Die Verpackungseinheit kann ferner in einen Gas-Temperierschrank einführbar sein, worin passende Bedingungen hinsichtlich Temperatur, Druck, Gaszusammensetzung und Feuchte herstellbar sind. Sofern eine Autonomie der Verpackungseinheit gewünscht wird, kann diese eine Regelvorrichtung umfassen, mittels welcher der physikalische Parameter regelbar ist oder geregelt wird.

Die Verpackungseinheit kann Mittel zum drahtlosen, vorzugsweise optischen oder funkgebundenen Übertragen von Informationen umfassen. Die Informationen können den physikalischen Parameter oder eine Identität des pharmazeutischen Objekts betreffen. Die Identität kann einen Produktnamen, einen Hersteller, eine Menge, ein Herstellungsdatum, eine Prozessierungsfolge oder ein Verfallsdatum, etc. betreffen. Die Informationen können an eine Bedienperson oder einen Empfänger in dem Außenbereich der Verpackungseinheit übermittelt werden.

Das Mittel zum drahtlosen Übertragen von Informationen kann i) auf RFID- oder RuBee- Technologie basieren, oder ii) ein elektronisches Display oder einen beschriftbaren Aufkleber umfassen. Im Fall ii) können die Informationen im Display angezeigt werden oder von einer Person auf den Aufkleber aufgetragen werden. Im Fall i) können die Informationen an einen Empfänger übertragen werden, zum Steuern oder Regeln des physikalischen Parameters oder zum Informieren einer Person.

Vorzugsweise kann ein Umströmen des Außenbereichs mit dem zweiten Sterilisationsmittel, für welches das Wandmaterial undurchlässig ist, ausgeführt werden, zum Sterilisieren des Außenbereichs. Das Umströmen des Außenbereichs kann
- mit einem Wasserstoffperoxiddampf ausgeführt werden, der vorzugsweise aus einer wässrigen Wasserstoffperoxid-Lösung, insbesondere in einer Konzentration > 5% und < 50%, mittels Verdampfen erzeugt wird, oder
- mit einem Gemisch aus Wasserstoffperoxiddampf und Luft ausgeführt werden, das vorzugsweise mindestens >5% und < 50% Wasserstoffperoxid enthält.

Ein Verschließen der Sterilisationskammer gegenüber dem Außenbereich kann ausgeführt werden, insbesondere mittels i) eines Verschlusses zum Verschließen einer Öffnung zwischen Sterilisationskammer und Vorkammer, oder ii) Versiegeln eines das Wandmaterial mit selektiver Durchlässigkeit für Sterilisationsmittel umfassenden Wandungsbereichs, vorzugsweise durch einen Aufkleber aus einem Metall, beispielsweise Aluminium. Das Verschließen mittels Metall-Aufkleber kann alternativ zum Verschließen mittels des zweiten Verschlusses umgesetzt werden.

Das Versiegeln der Vorkammer gegenüber der Sterilisationskammer oder dem Außenbereich kann ausgeführt werden mittels Verschweißen, vorzugsweise Thermoschweißen, Heißschweißen, Hochfrequenzschweißen oder Mikrowellenschweißen, der entsprechenden Öffnungen. Hierbei können Bereiche der Beutel-Folie miteinander verschweißt werden.

Die Erfindung wird nachfolgend anhand von Ausführungsbeispielen und unter Bezugnahme auf die Zeichnungen näher erläutert. Dabei verweisen gleiche Bezugszeichen auf gleiche oder entsprechende Elemente. Die Merkmale verschiedener Ausführungsbeispiele können miteinander kombiniert werden.

### Kurzbeschreibung der Figuren

Es zeigen:
- Fig. 1: eine Verpackungseinheit gemäß dem Stand der Technik,
- Fig. 2a-d: Arbeitsphasen für eine erfindungsgemäße Verpackungseinheit gemäß einer ersten Ausführungsform,
- Fig. 3: eine erfindungsgemäße Verpackungseinheit gemäß einer zweiten Ausführungsform, und
- Fig. 4a, 4b: erfindungsgemäße Verpackungseinheiten gemäß einer dritten Ausführungsform, mit unterschiedlichen pharmazeutischen Objekten.

### Detaillierte Beschreibung der Erfindung

Die Vorgabe der Erfindung liegt darin, eine aus dem Stand der Technik bekannte Verpackungseinheit 100 (siehe Fig. 1) dahingehend zu ändern, dass sie eine hohe Sterilität des verpackten Objekts ermöglicht und zugleich einfach in der Bedienung ist. Die Idee der Erfindung, eine Mehrkammer-Struktur 14, 16 zu verwenden, wobei eine Wandung einer der Kammern bereichsweise ein Wandmaterial 18 mit selektiver Durchlässigkeit für Sterilisationsmittel 24, 26 umfasst löst auf elegante Weise die Aufgabe der Erfindung, indem das Sterilisieren als eine Reduktion der Anzahl von Mikroorganismen von vorzugsweise mindestens vier Zehnerpotenzen aufgefasst wird. Die erfindungsgemäße Verpackungseinheit 10 ermöglicht eine zweistufige Reduktion der Anzahl von Mikroorganismen: i) mittels Sterilisieren, wobei Mikroorganismen weitgehend abgetötet werden, und ii) mittels Evakuieren, wobei ein Großteil der übriggebliebenen Mikroorganismen mit dem Gas abgepumpt werden. Durch das Evakuieren wird die Dichtheit des Beutels auf einfachere Weise indiziert.

Die Umsetzung einer Sterilisierung mit Hilfe der Verpackungseinheit 10 wird anhand der Fig. 2a - 2d nachfolgend erläutert. Diese Figuren zeigen die Verpackungseinheit 10 in strukturell-funktionalen, jedoch nicht naturalistischen Zeichnungen.

Fig. 2a zeigt die erfindungsgemäße Verpackungseinheit 10 für ein pharmazeutisches Objekt 12. Die Verpackungseinheit 10 umfasst i) eine Sterilisationskammer 14 zum Aufnehmen des Objekts 12 und ii) eine Vorkammer 16, die zu einem Außenbereich der Verpackungseinheit 10 und zu der Sterilisationskammer 14 hin jeweils verschließbar geöffnet ist. Eine Wandung der Vorkammer 16 umfasst bereichsweise ein Wandmaterial 18 mit selektiver Durchlässigkeit für Sterilisationsmittel 24, 26.

Das Wandmaterial 18 mit selektiver Durchlässigkeit für Sterilisationsmittel 24, 26 ist ein textiler Stoff, vorzugsweise ein Vliesstoff aus Kunststoff-Fasern, vorzugsweise ein Polyethylenfasern-Vlies. Als Beispiel sei Tyvek® als geeigneter Vliesstoff genannt. Die Verpackungseinheit 10 ist als ein Kunststofffolien-Beutel, beispielsweise als PVC-Beutel ausgebildet, der erste und der zweite Verschluss 20, 22 sind als Ziploc-Verschlüsse ausgebildet. Die Verschlüsse 20, 22 sind offen, so dass das pharmazeutische Objekt 12 in die Sterilisationskammer 14 eingeführt werden kann. Das erste Sterilisationsmittel 24 umfasst ETO, das zweite Sterilisationsmittel 26 umfasst VHP.

In Fig. 2a, worin alle Elemente der Verpackungseinheit 10 erkennbar sind, befindet sich das pharmazeutische Objekt 12 außerhalb der Verpackungseinheit 10. Das ist ein Zustand vor Beginn der Sterilisation.

Fig. 2b zeigt die Verpackungseinheit 10 in welche das pharmazeutische Objekt 12 bereits eingeführt wurde; dieses befindet sich in der Sterilisationskammer 14. Der erste Verschluss 20 ist bereits gasdicht verschlossen, der zweite Verschluss 22 ist geöffnet. Die Sterilisation wird durchgeführt durch das Einleiten des ersten Sterilisationsmittels 24, für welches das Wandmaterial 18 durchlässig ist, durch das Wandmaterial 18 hindurch in einen Innenbereich der Verpackungseinheit 10, zum Sterilisieren des Innenbereichs und des Objekts 12. Anschließend kann durch das Umströmen des Außenbereichs mit dem zweiten Sterilisationsmittel 26, der Außenbereich sterilisiert werden. Vorzugsweise kann dazu die erste Kammer verschlossen werden.

Alternativ zum Sterilisieren des Außenbereichs mit dem ersten Sterilisationsmittel 24 kann eine Entkeimung des Außenbereichs mit UV-C Licht, Gammastrahlen oder Heißluftsterilisation durchgeführt werden.

Die genannte Reihenfolge (erstes Sterilisationsmittel 24 und dann zweites Sterilisationsmittel 26) kann auch umgekehrt werden, indem zuerst das zweite Sterilisationsmittel 26 und danach das erste Sterilisationsmittel 24 eingeleitet werden. Die Verpackungseinheit 10 kann auch gleichzeitig mit beiden Sterilisationsmitteln umströmt werden, denn in jedem Fall dringt das erste Sterilisationsmittel 24 in die Verpackungseinheit 10 ein und die Bakterien und Mikroorgansimen werden blockiert.

In Fig. 2c ist der zweite Verschluss 22 geschlossen, der erste Verschluss 10 ist ebenfalls geschlossen. Der Zustand (geöffnet oder geschlossen) des ersten Verschlusses 10 ist in dieser Phase allerdings nicht relevant, da der erste Verschlusses 10 zur Dichtigkeit der Sterilisationskammer 14 gegenüber dem Außenbereich keinen Beitrag leistet; die Dichtigkeit ist durch das Schließen des zweiten Verschlusses 22 hergestellt. In dieser Konfiguration wird das Evakuieren der Sterilisationskammer 14 auf einen Druck von < 200 mbar durchgeführt, mittels Abpumpen über einen hier nicht gezeigten Vakuumport der Sterilisationskammer 14. Nach Beendigung der Evakuierung ist das Sterilisieren des pharmazeutischen Objekts 12 und der Sterilisationskammer 14 beendet.

Fig. 2d zeigt eine alternative Möglichkeit, eine Dichtigkeit der Sterilisationskammer 14 gegenüber dem Außenbereich herzustellen: der Bereich des Wandmaterials 18 mit selektiver Durchlässigkeit wird mit einem Verschlussaufkleber 30 luftdicht verschlossen. Auch in dieser Konfiguration kann das Evakuieren der Sterilisationskammer 14 durchgeführt werden.

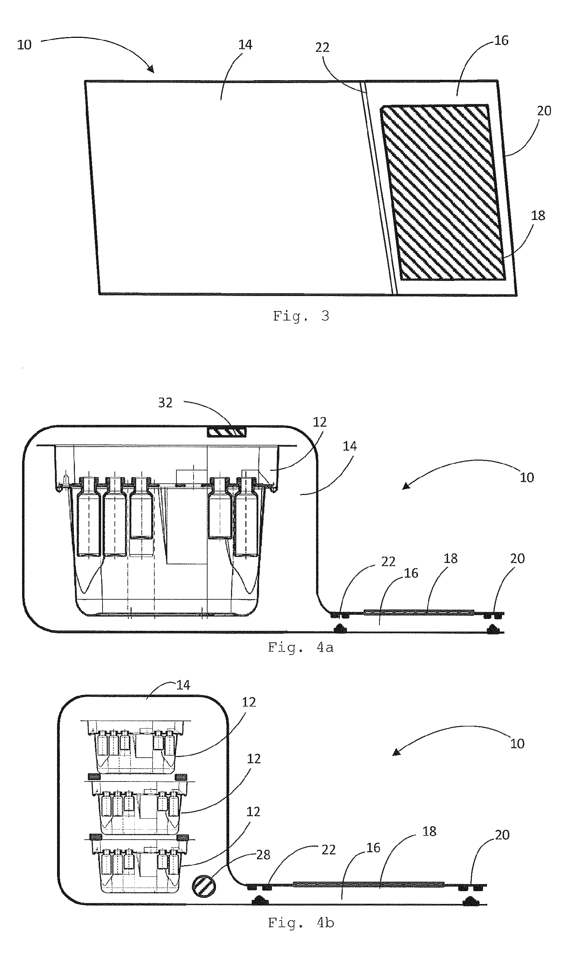

Fig. 3 zeigt die Verpackungseinheit 10 in einer naturalistischen Zeichnung, in einer Seitenansicht, wobei in dem Innenbereich der Verpackungseinheit 10 kein pharmazeutisches Objekt angeordnet ist. Hier ist erkennbar, dass die Verpackungseinheit 10 als ein Beutel ausgebildet ist.

Fig. 4a, 4b zeigen die Verpackungseinheit 10 jeweils in einer Seitenansicht, wobei ein oder mehrere pharmazeutische Objekte 12 in der Sterilisationskammer 14 angeordnet sind.
In Fig. 4a ist das pharmazeutische Objekt 12 ein Behälternest oder Tub für mehrere Behälter, die als Medikamentenbehälter ausgebildet sind. In Fig. 4b ist das pharmazeutische Objekt 12 gebildet aus einer Mehrzahl aufeinandergestapelten Behälterneste. In dieser Figur ist ein Sensor 32 erkennbar, mittels welchem zumindest ein physikalischer Parameter im Inneren der Verpackungseinheit, insbesondere eine Temperatur, Gaszusammensetzung, Feuchte, elektromagnetische Strahlung oder ein Druck, messbar ist. Die Daten des Sensors werden mittels eines RFID- oder RuBee- Chips 32 nach außen übertragen.

### Ergänzende Darstellung der Erfindung

Die Erfindung stellt einen gasdichten, wiederverschließbaren Beutel 10 für Transportbehälter 12 dar für Fläschchen (Vials), Spritzen (syringe), Doppelkammer-Spritzen (dual-chamber syringe), Karpullen (cartridges), Doppelkammer-Karpulen (dual chamber cartridges), sowie Vartridges, die flexibel genug sind um unterschiedliche Größen (Länge und Durchmesser) sicher zu transportieren und die es ermöglichen die Pharmaverpackungen, den Transportbehälter und den Beutel keimfrei zu realisieren. Die Verpackung kann verwendet werden für kosmetische, medizinische oder pharmazeutische Anwendungen. Der Beutel 10 kann vollständig dekontaminiert werden, sowohl im Hinblick auf die äußere Oberfläche, die innere Oberfläche, das Innere des Behälters und auf die Füllung.

Die Erfindung stellt einen wieder verschließbaren gasdichten Beutel 10 für den Transport (innerhalb und außerhalb des Produktionsprozesses) von Transportbehältern 12 mit pharmazeutischen Behältern bereit, der innen mit ETO und außen mit VHP desinfizierbar ist, ohne dass die Behälter VHP kontaminiert werden. Der Beutel ist evakuierbar und mit Sensoren ausgestattet, die das Vakuum und weitere Parameter wie z.B. die Temperatur kontrollieren. Die vorliegende Erfindung bezieht sich auf die innere und äußere Dekontamination eines an sich bekannten eingepackten Gegenstands insbesondere im medizinischen und pharmazeutischen Bereich.

Der Beutel 10 ermöglicht es Transportbehälter 12 für Fläschchen (Vials), Spritzen (syringe) und Doppelkammer-Spritzen (dual-chamber syringe) oder Karpullen (cartridges) und Doppelkammer-Karpulen (dual chamber cartridges), sowie Vartridges, mit unterschiedlichen Größen (Länge und Durchmesser) sicher und keimfrei zu transportieren. Der Beutel 10 kann verwendet werden für kosmetische, medizinische oder pharmazeutische Anwendungen.

Der Beutel 10 kann mehrfach mit einem Druckverschluss 20, 22 oder Zipper verschlossen werden. Der Beutel 10 lässt sich mehrfach luftdicht verschließen und kann die Behälter steril aufbewahren. Die Verpackung kann mit einer Tyvek Folie 18 versehen werden um eine Sterilisierung in der Verpackung zu ermöglichen. Der Beutel 10 kann hinter dem Druckverschluss/Zipper 20, 22 mittels Thermoschweißen, Heißschweißen, Hochfrequenzschweißen und/oder Mikrowellenschweißen versiegelt werden.

Der Beutel 10 und/oder die Verpackung kann mit einem Fluoreszenzplagiatschutz oder mit einem RFID/RuBee Chip mit einem Sensor (beispielsweise 02, Feuchtigkeit, Temperatur) versehen werden. Der Beutel 10 kann äußerlich keimfrei realisiert werden.

Die vorliegende Erfindung betrifft allgemein die sterile Verpackung einer Mehrzahl von Behältern zur Aufbewahrung von Substanzen für medizinische, pharmazeutische oder kosmetische Anwendungen, insbesondere von Fläschchen, Ampullen oder Karpulen, und betrifft insbesondere eine Verpackungsstruktur mit Beutel, in der Behälter in einfacher Weise sterilisiert werden können und anschließend sicher transportiert werden können.

Gemäß einem Verfahren zur sterilen Verpackung von Behältern mit einem Beutel 10 für medizinische, pharmazeutische oder kosmetische Anwendungen können folgende Schritte ausgeführt werden: Bereitstellen eines Trägers 12, Anordnen der Mehrzahl von Behältern in den Aufnahmen des Trägers 12; Bereitstellen eines Beutels 10, der zumindest einen gasundurchlässigen Abschnitt 18 aufweist, um die Aufnahmen steril zu verpacken; Verbinden der Öffnung der Schutzfolie mit einem Zipperverschluss 20 und wenn nötig mit einer Klammer um die Verpackungsstruktur wie vorstehend beschrieben auszubilden.

Gemäß einer Ausführungsform können die Aufnahmen nachträglich durch Einströmen eines ETO-Gases durch zumindest einen gasdurchlässigen Abschnitt (Tyvek®) 18 des Beutels 10 sterilisiert bzw. entkeimt werden.

Gemäß einer Ausführungsform ist der Beutel 10 aus einem Material hergestellt, welches Wasserstoffperoxid (H2O2) Dampf (VHP) resistent ist. Geeignet sind besondere chemikalienresistente Kunststoffe, wie beispielsweise Polyamid (PA), Polyethylen (PE), Polycarbonat (PC), Polypropylen (PP), PSU und PVC. Geeignet ist auch eine Metallfolie oder Aluminiumfolie. Besonders geeignet ist ein Aluminium-Polypropylen-Verbundmaterial als Folie. Wasserstoffperoxyd (VHP) wirkt entkeimend, kann in vorteilhaft einfacher und kostengünstiger Weise durch aktives Verdampfen einer wässrigen Wasserstoffperoxid-Lösung erzeugt werden und so zum Sterilisieren des Außenbereiches des Beutels 10 verwendet werden. Um eine hohe biologische Dekontaminationsrate der Mikroorganismen zu erreichen, ist eine definierte hohe Konzentration von >5% bis 50% erforderlich. Die Entkeimung kann auch mit UVC-Licht oder Gammastrahlen erfolgen. Notwendig ist es den Tyvek®-Bereich 18 mit dem restlichen Beutelabschnitt durch einen zweiten Zippverschluss 22 und/oder einer Klammer und/oder durch Thermoschweißen zu verschließen. Allgemein wirkt Wasserstoffperoxid zytotoxisch und durch seine starke Toxizität gegenüber vielen prokaryotischen Kleinstlebewesen desinfizierend. Nach der VHP Dekontamination, beträgt die Wasserstoffperoxyd-Konzentration in den Behältern die sich innerhalb des Beutels in einem Transportbehälter befinden vorzugsweise maximal 0.03 ppm.

Gemäß einem Verfahren zur Bearbeitung oder Verarbeitung von Behältern für medizinische, pharmazeutische oder kosmetische Anwendungen unter Verwendung eines Beutels 10 und Verpackungsstruktur, wie vorstehend beschrieben, können folgende Schritte ausgeführt werden:
- Bereitstellen und Öffnen eines Beutels 10;
- Aufnahme von mindestens einem Transportbehälter 12 (mehrere sind auch möglich) mit einem Nest mit einer Mehrzahl von Aufnahmen, in denen jeweils Behälter aufgenommen sind;
- Verschließen des Beutels 10, bevorzugt steril;
- Begasen des Inneren des Beutels 10 mit ETO durch einen gasdurchlässigen Abschnitt (Tyvek®) 18, dessen Blockierungsschwelle jedoch so bestimmt ist, dass es den Durchtritt von Kontaminanten ins Innere der Umhüllung aufhält;
- Verschließen des Beutels hinter dem Tyvek-Abschnitt 18.

Alternativ kann das Tyvek-Fenster 18 durch einen "Alu-Aufkleber" 30 versiegelt werden, und/oder durch eine Klammer und/oder durch Thermoschweißen, oder Heißschweißen, oder Hochfrequenzschweißen oder Mikrowellenschweißen geschlossen werden.
- Optionale Evakuierung des Beutel (< 200 mbar);
- Dekontaminierung des äußeren Beutels mit VHP;
- Optionales Verpacken mit einem zweiten gasdichten Beutel.

Innerhalb des Beutels oder innerhalb der Transportbehälter im Beutel kann mindestens ein elektronisch drahtlos (wireless) auslesbarer RFID-Chip oder RuBee-Chip angeordnet sein (ein RuBee Chip funkt auf Frequenzen, die Metall- und Wasser durchdringen können), der durch die Seitenwände der Verpackungseinheit hindurch berührungslos ausgelesen werden kann und auf Abfrage Information bezüglich der Identität, wichtiger Produkteigenschaften (Hersteller, Inhalt, Herstellungsdatum, Verfallsdatum, ...) etc. ausgibt. Der Chip kann in die Verpackungseinheit 10 an geeigneter Stelle eingeklebt oder verschweißt sein.

Gemäß einer Ausführungsform kann der RuBee- oder RFID Chip in Kombination mit weiteren Sensoren integriert werden, die Parameter des Transport- und Verpackungsbehälters zeitabhängig überwachen können, welche Qualitäts- oder Echtheitseigenschaften der in dem Transport- und Verpackungsbehälter aufbewahrten Behälter betreffen. Diese Qualitäts- oder Echtheitseigenschaften können periodisch aufgezeichnet und in einem dem Chip oder Sensor zugeordneten Speicher abgelegt werden. Zur elektrischen Stromversorgung dieser elektronischen Bauelemente kann in dem Transport- und Verpackungsbehälter eine netzunabhängige Spannungsversorgung vorgesehen sein, insbesondere eine Batterie mit geringen Abmessungen oder auch induktiv über eine kleine Leiterschleife. Als Sensoren sind gemäß der vorliegenden Anmeldung insbesondere angedacht:
- ein Feuchtigkeitssensor mit oder ohne Speicher (Data-Logging), der die in dem Transport- und Verpackungsbehälter vorherrschende Luftfeuchtigkeit periodisch misst und bei Bedarf aufzeichnet;
- ein Gassensor mit oder ohne Speicher (Data-Logging), der die Konzentration von in dem Transport- und Verpackungsbehälter vorhandenen Gasen, wie beispielsweise 02, Ozon, CO2 oder von Entkeimungsgase, wie z.B. Ethylenoxyd, Formaldehyd, misst und bei Bedarf aufzeichnet;
- ein Temperatursensor mit oder ohne Speicher (Data-Logging), der die in dem Transport- und Verpackungsbehälter vorherrschende Temperatur periodisch misst und bei Bedarf aufzeichnet;
- ein UV Sensor mit oder ohne Speicher (Data-Logging), der in den Transport- und Verpackungsbehälter eindringende UV-Strahlung periodisch misst und bei Bedarf aufzeichnet;
- ein Gammastrahlungs-, Elektronenstrahlen- oder Röntgenstrahlensensor mit oder ohne Speicher (Data-Logging), der in den Transport- und Verpackungsbehälter eindringende Strahlung periodisch misst und bei Bedarf aufzeichnet;
- ein Drucksensor mit oder ohne Speicher (Data-Logging), der den Druck von den in dem Transport- und Verpackungsbehälter vorhandenen Gases misst und bei Bedarf aufzeichnet.

Die Gas- oder Drucksensoren können beispielsweise zum Nachweis der Unversehrtheit der Verpackungseinheit und der darin aufgenommenen Behälter dienen.

Die Erfindung stellt also einen wiederverschließbaren gasdichten Beutel 10 für den Transport von Transportbehältern 12 mit pharmazeutischen Behältern bereit, der innen mit ETO und außen mit VHP desinfizierbar ist, ohne dass die Behälter VHP kontaminiert werden. Der Beutel 10 ist evakuierbar und mit Sensoren 32 ausgestattet, die das Vakuum und z.B. die Temperatur messen.

Die Fig. 3 zeigt einen Beutel 10 mit zwei Zip-Verschlüssen 20, 22 und einem gasdurchlässigen Abschnitt 18 (Tyvek® Folie) auf. Der Beutel 10 weist mindestens einen gasdurchlässigen Abschnitt 18 auf, der beidseitig gasdicht wiederverschließbar ist. Dazu kann ein Druck- oder Zipverschluss verwendet werden. Durch den Tyvek®-Bereich kann der Inhalt des Beutels mit Gas (z.B. ETO) entkeimt werden. Die wiederverschließbaren Beutel können aus Polyethylen LD (LDPE/ PE-LD), Aluminium oder z.B. Polypropylen (PP) hergestellt werden. Aber auch MDPE, HDPE, COEX, Verbundfolien mit Regeneratanteilen sind möglich. Eine farbige Einfärbung kann Vorteile bieten (Solarisation, Markierung, Chargencodierung). Auch kann die Färbung blickdicht gestaltet sein.

Die Fig. 4a zeigt in einer Schnittansicht einen Beutel 10, mit Transportbehälter 12 (Nest, Tub und pharmazeutischen Behältern/Fläschchen), mit einem Sensor und einem RFID oder RuBee Chip 32, mit zwei Zip-Verschlüssen 20, 22 und einem gasdurchlässigen Abschnitt 18 (Tyvek® Folie) auf. Ein erster Druckverschluss 20 ist verschlossen, so dass Gas nur durch den gasdurchlässigen Tyvek® Bereich einströmen kann. Der zweite Druckverschluss 22 kann so gestaltet sein, dass der untere Bereich gebogen ist, so dass die Gaseinströmung erleichtert wird.

Die Fig. 4b zeigt in einer Schnittansicht einen Beutel, mit drei Transportbehältern 12 (Nest, Tub und pharmazeutischen Behältern/Fläschchen), mit zwei Zip-Verschlüssen 20, 22 und einem gasdurchlässigen Abschnitt 18 (Tyvek® Folie) auf. Die Druckverschlüsse sind noch nicht verschlossen. Der Beutel 10 enthält einen verschließbaren "Vakuumport", durch den der Beutel 10 evakuiert werden kann.

### Bezugszeichen

- 100: Schieberbeutel, Verpackungseinheit gemäß Stand der Technik
- 102: Schieber-Reißverschluss
- 104: Öffnung
- 106: Aufkleber
- 10: Verpackungseinheit, Beutel
- 12: pharmazeutisches Objekt
- 14: Sterilisationskammer
- 16: Vorkammer
- 18: Wandmaterial mit selektiver Durchlässigkeit für Sterilisationsmittel
- 20: erster Verschluss
- 22: zweiter Verschluss
- 24: erstes Sterilisationsmittel
- 26: zweites Sterilisationsmittel
- 28: Vakuumport
- 30: Verschlussaufkleber
- 32: Sensor mit RFID/RuBee

## Patentansprüche

1. Verpackungseinheit (10) für mindestens ein pharmazeutisches, medizinisches oder kosmetisches Objekt (12), umfassend:
eine Sterilisationskammer (14) zum Aufnehmen des Objekts (12) und
eine Vorkammer (16), die zu einem Außenbereich der Verpackungseinheit (10) und zu der Sterilisationskammer (14) hin jeweils verschließbar ist,
wobei eine Wandung der Vorkammer (16) bereichsweise ein Wandmaterial (18) mit selektiver Durchlässigkeit für Sterilisationsmittel (24, 26) umfasst, zum Ermöglichen des Sterilisierens eines Innenbereichs der Verpackungseinheit (10) oder des Objekts (12).

2. Verpackungseinheit (10) gemäß Anspruch 1, **gekennzeichnet durch** mindestens eines der folgenden Merkmale:
das selektiv durchlässige Wandmaterial (18) ist für ein, insbesondere gasförmiges, erstes Sterilisationsmittel (24) durchlässig, wobei vorzugsweise das erste Sterilisationsmittel (24) **durch** das Wandmaterial (18) in einen Innenbereich der Verpackungseinheit (10) zum Sterilisieren oder Desinfizieren oder Dekontaminieren des Innenbereichs einleitbar ist;
das selektiv durchlässige Wandmaterial (18) ist für Mikroorganismen undurchlässig;
das erste Sterilisationsmittel (24) umfasst ein als ETO bezeichnetes Ethylenoxid;
das selektiv durchlässige Wandmaterial (18) umfasst einen Vliesstoff;
der Vliesstoff ist als Tyvek® ausgebildet.

3. Verpackungseinheit (10) gemäß einem der Ansprüche 1 oder 2, **gekennzeichnet durch** mindestens eines der folgenden Merkmale:
die Vorkammer (16) weist einen ersten, insbesondere gasdichten, Verschluss (20) zum, vorzugsweise öffnenbaren, Verschließen der Öffnung zu dem Außenbereich;
die Vorkammer (16) weist einen zweiten, insbesondere gasdichten, Verschluss (22) zum, vorzugsweise öffnenbaren, Verschließen der Öffnung zu der Sterilisationskammer (14);
der erste oder zweite Verschluss (20, 22) ist von außen betätigbar zum Schließen oder Öffnen der entsprechenden Öffnungen;
der erste oder zweite Verschluss (20, 22) ist als ein Druckverschluss oder Reißverschluss, vorzugsweise als ein einfacher oder mehrfacher Verschluss, ausgebildet.

4. Verpackungseinheit (10) gemäß einem der vorstehenden Ansprüche, **gekennzeichnet durch** mindestens eines der folgenden Merkmale:
die Verpackungseinheit (10) ist als ein Beutel ausgebildet;
eine Wandung der Verpackungseinheit (10) ist resistent gegen VHP oder ETO;
die Wandung der Verpackungseinheit (10) umfasst einen chemikalienresistenten Kunststoff, vorzugsweise ein als PA bezeichnetes Polyamid oder ein als PE bezeichnetes Polyethylen oder ein als PC bezeichnetes Polycarbonat oder ein als PP bezeichnetes Polypropylen oder ein als PSU bezeichnetes Polysulfon oder ein als PVC bezeichnetes Polyvinylchlorid;
die Wandung der Verpackungseinheit (10) umfasst eine Folie aus einem Metall, vorzugsweise Aluminium, oder aus einem Aluminium-Polypropylen-Verbundmaterial.

5. Verpackungseinheit (10) gemäß einem der vorstehenden Ansprüche, **gekennzeichnet dadurch, dass** die Verpackungseinheit (10), insbesondere die Sterilisationskammer (14), evakuierbar ist, wobei vorzugsweise ein im Inneren der Verpackungseinheit (10) oder der Sterilisationskammer (14) vorhandenes Gas abpumpbar ist zum Reduzieren eines Drucks im Inneren der Verpackungseinheit (10) oder der Sterilisationskammer (14).

6. Verpackungseinheit (10) gemäß einem der vorstehenden Ansprüche, **gekennzeichnet durch** mindestens eines der folgenden Merkmale:
zumindest ein physikalischer Parameter im Inneren der Verpackungseinheit (10), insbesondere eine Temperatur, Gaszusammensetzung, Feuchte, elektromagnetische Strahlung, oder ein Druck, ist messbar, vorzugsweise mittels zumindest eines in der Verpackungseinheit (10) angeordneten Sensors;
die Verpackungseinheit (10) umfasst Mittel zum Ändern des physikalischen Parameters;
die Verpackungseinheit (10) umfasst eine Regelvorrichtung, mittels welcher der physikalische Parameter regelbar ist;
die Verpackungseinheit (10) umfasst Mittel zum drahtlosen, vorzugsweise optischen oder funkgebundenen Übertragen von Informationen, betreffend insbesondere den physikalischen Parameter oder eine Identität des pharmazeutischen Objekts (12), an eine Bedienperson oder einen Empfänger in dem Außenbereich der Verpackungseinheit (10);
das Mittel zum drahtlosen Übertragen von Informationen i) basiert auf RFID- oder RuBee- Technologie, oder ii) umfasst ein elektronisches Display oder einen beschriftbaren Aufkleber.

7. Verpackungseinheit (10) gemäß einem der vorstehenden Ansprüche, mit einem pharmazeutischen, medizinischen oder kosmetischen Objekt (12), welches als ein Behälter oder ein Verpackungsbehälter, Behälternest oder Tub für mindestens einen Behälter ausgebildet ist.

8. Verwendung einer Verpackungseinheit (10) gemäß einem der Ansprüche 1 bis 6 zum Sterilisieren und sterilen Transportieren eines pharmazeutischen, medizinischen oder kosmetischen Objekts (12), welches als i) ein Behälter oder ii) ein Verpackungsbehälter, Behälternest oder Tub für mindestens einen Behälter ausgebildet ist, wobei vorzugsweise der Behälter ein Medikamentenbehälter, ein Fläschchen, eine Ampulle, eine Karpule, eine Vartridge, eine Doppelkammer-Karpule, eine Spritze, oder eine Doppelkammer-Spritze ist.

9. Verfahren zum Sterilisieren eines in einer Verpackungseinheit (10) anordenbaren pharmazeutischen Objekts (12), die Verpackungseinheit (10) umfassend eine Sterilisationskammer (14) und eine mit der Sterilisationskammer (14) fluidverbundene Vorkammer (16), die zu einem Außenbereich der Verpackungseinheit (10) verschließbar geöffnet ist und eine Wandung aufweist, die bereichsweise ein Wandmaterial (18) mit selektiver Durchlässigkeit für Sterilisationsmittel (24, 26) umfasst, das Verfahren umfassend die Schritte:
Einbringen mindestens eines Objekts (12) in die Sterilisationskammer (14);
Verschließen der Vorkammer (16) gegenüber dem Außenbereich; und
Einleiten eines ersten Sterilisationsmittels (24), für welches das Wandmaterial (18) durchlässig ist, von dem Außenbereich durch das Wandmaterial (18) in einen Innenbereich der Verpackungseinheit (10), zum Sterilisieren des Innenbereichs oder des Objekts (12).

10. Verfahren gemäß dem vorstehenden Anspruch, umfassend mindestens einen der folgenden Schritte:
Umströmen des Außenbereichs mit einem zweiten Sterilisationsmittel (26), für welches das Wandmaterial (18) undurchlässig ist, zum Sterilisieren des Außenbereichs;
Umströmen des Außenbereichs mit einem Wasserstoffperoxiddampf, der vorzugsweise aus einer wässrigen Wasserstoffperoxid-Lösung, insbesondere in einer Konzentration > 5% und < 50%, mittels Verdampfen erzeugt wird;
Umströmen des Außenbereichs mit einem Gemisch aus Wasserstoffperoxiddampf und Luft, das vorzugsweise mindestens 5% und maximal 50% Wasserstoffperoxid enthält;
Verschließen der Sterilisationskammer (14) gegenüber dem Außenbereich, insbesondere mittels i) eines Verschlusses (22) zum Verschließen einer Öffnung zwischen Sterilisationskammer (14) und Vorkammer (16), oder ii) Versiegeln eines das Wandmaterial (18) mit selektiver Durchlässigkeit für Sterilisationsmittel (24, 26) umfassenden Wandungsbereichs, vorzugsweise durch einen Aufkleber (30) aus einem Metall, beispielsweise Aluminium;
Versiegeln der Vorkammer (16) gegenüber der Sterilisationskammer (14) oder dem Außenbereich mittels Verschweißen, vorzugsweise Thermoschweißen, Heißschweißen, Hochfrequenzschweißen oder Mikrowellenschweißen der entsprechenden Öffnungen;
Evakuieren eines Innenbereichs der Verpackungseinheit (10), vorzugsweise der Sterilisationskammer (14), insbesondere auf einen Druck von < 200 mbar.

11. Verfahren gemäß einem der vorstehenden zwei Ansprüche, umfassend mindestens einen der folgenden Schritte:
Messen zumindest eines physikalischen Parameters im Inneren der Verpackungseinheit (10), insbesondere einer Temperatur, eines Drucks, einer Gaszusammensetzung, einer elektromagnetischen Strahlung oder einer Feuchte;
Regeln des physikalischen Parameters im Inneren der Verpackungseinheit (10).

12. Verfahren gemäß einem der vorstehenden drei Ansprüche, umfassend mindestens einen der folgenden Schritte:
Verpacken mit einem zweiten gasdichten Beutel;
Messen zumindest eines physikalischen Parameters im Inneren der zweiten Verpackungseinheit, insbesondere einer Temperatur, eines Drucks, einer Gaszusammensetzung, einer elektromagnetischen Strahlung oder einer Feuchte.
